# EUROPEAN PATENT APPLICATION

(11) **EP 1 005 864 A1**
(43) Date of publication of application: **07.06.2000**
(21) Application number: 98670006.0
(22) Date of filing: 30.11.1998
(51) Int. Cl.: A61K 31/435, A61K 9/20, A61K 9/28

(54) **Antioxidant free stable pharmaceutical compositions containing thienopyridine derivatives**

(71) Applicant: TECNIMEDE-SOCIEDADE TECNICO-MEDICINAL, S.A., 2685 Prior Velho (PT)
(72) Inventor: Nunes Da Costa Gomes Sobral Da Silva,Maria Jacinta, Linda A Velha (PT); Sousa Goucha Jorge, Pedro Manuel, 2670 Loures (PT); Martins Peres Alves, Rui Manuel, 2750 Cascais (PT)
(74) Representative: Schlee, Alexander, Dipl.-Ing.

(57) **Abstract**

The present invention relates to a stable pharmaceutical composition containing at least a thienopyridine derivative acid salt as the active ingredient, namely 5-(2-chrobenzyl)-4,5,6,7-tetrahydrothieno[3, 2-c]-pyridine hydrochloride, and an excipient not including the active ingredient degradation.

## Description

### INVENTION FIELD

The present invention relates to pharmaceutical compositions that contain acid salts of thienopyridine derivatives and are stabilized without having recourse to any antioxidant agent.

### INVENTION BACKGROUND

### Oxidative Decomposition

The oxidative decomposition of pharmaceutical compounds is responsible for the instability of a great number of pharmaceutical preparations. These reactions are mediated by free radicals or by molecular oxygen. The more general form of oxidative decomposition occurring in pharmaceutical compositions is a self-oxidation process involving a free radical mechanism of chain reactions. In general, a self-oxidation may be defined as the reaction of any compound with molecular oxygen.

Free radicals are produced by reactions involving rupture of a covalent bond in such a way that each involved atom or group retains one of the electrons of the original covalent bond. These radicals are strongly insaturated and quickly gain electrons from other compounds and cause their oxidation. The starting of the reaction may be produced by thermal decomposition of the compounds which are present or are added or by light. The reaction may be stopped by combining two different radicals or by means of a free radical inhibitor such as sodium metabisulfite. In the self oxidative reactions, only a very little amount of oxygen is necessary to start the reaction and therefore the oxygen concentration will be not important. The heavy metals, particularly those having two or more oxidation states and distant redox potentials, usually catalyse the oxidative deteriorations. Their main function is to increase the free radical formation rate.

### Antioxidants

Antioxidants make a group of excipients that are used in medicaments in order to decrease the oxidation of active compounds and/or excipients in a ready product. Three types of antioxidantes are known:
- Real antioxidants - they block the chain reaction reading with the free radicals and forming stable or metastable products and stopping the oxidation reaction;
- Reducing agents - they have a lower oxidation potential than the active compound or excipient they protect, being therefore preferably oxidised;
- Synergistical antioxidants - they have a small antioxidant effect in themselves, but they increase the action of other antioxidants because they form complexes with the heavy metal ions which otherwise would catalyse the oxidation.

Examples of chelant agents are ethylenediaminetetraacetic acid (EDTA) derivatives and salts and citric and tartaric adds.

### Thienopyridines

Thienopyridines, particularly Ticlopidine, have inhibitory properties at the level of certain plateletary and erythrocitary functions and can interfere in the arterial and veiny thrombosis since they decrease the platelet aggregation.

Due to the possibility of suffering oxidation processes implying a therapeutical potential decrease, some compositions have been prepared which contain antioxidant agents with the aim of protecting the molecule against the oxidative degradation.

The compositions containing Ticlopidine hydrochloride as described by U.S. Patent No. 4,051,141, are liable to a discoloration process due to Ticlopidine degradation during the storage period.

Portuguese Patent No. 73442 describes pharmaceutical compositions containing acid salts of thienopyridine derivatives which are stabilized by antioxidant and chelating agents, namely, non volatile and non toxic acids having a pKa of 2-6. However, the prevention of the ready product discoloration will be not the only reason for including antioxidant agents in a formulation.

Recently, the European Agency for Evaluating Medicinal Products published guidelines about antioxidant use, in the publication *"Note for Guidance on Inclusion of Antioxidant and Antimicrobial Preservatives in Medicinal Products".* (CPMP/CVMP/QWP/115/95) that took effect in January 1998.

According to this document, the use of antioxidants must be restrained taking in consideration both following principles:
1. *Antioxidants should not be used to mask unsatisfactorily formulated or packed products.*
2. *Antioxidants must be included in a formulation if it is proofed that their use can not be avoided.*

Therefore, the accomodation of the pharmaceutical compositions containing antioxidants to said guideline *"Note for guidance or inclusion of Antioxidantes and Antimicrobial Preservatives in Medicinal Products"* (CPMP/CVMP/QWP/115/95) requires the demonstration that the oxidation factors can not be eliminated.

### DETAILED DISCLOSURE OF THE INVENTION

The preparation of pharmaceutical compositions with the purpose of providing an easy administration way for an active compound accurate dose, nearly always requires the use of adjuvants having the functions of making feasible the industrial production, providing stability to the active compound during a tong period of time and therefore ensuring the maintenance of the therapeutic effect, and making easier the availability of the active compound to the necessary site. These adjuvants, generally called excipients, as a general rule, are inert and unprovided of therapeutical activity; therefore, their amounts preferably have to be not higher than the necessary to obtain the desired effects.

The aim of the development work to obtain the pharmaceutical compositions that, are the object of this invention was the confirmation of the oxidation factors for the thienopyridine derivatives and, afterwards, to withdraw them from the formulation so that stable compositions are obtained without adding any antioxidant and the previously mentioned guideline is satisfied *(Note for Guidance on Inclusion of Antioxidantes and Antimicrobial Preservatives in Medicinal Products* - CPMP/CVMP/QWP/115/95). This methodology makes possible avoiding the use of antioxidants solely for masking the presence of oxidation factors, a common characteristic of prior art formulations. The pharmaceutical compositions of the present invention are therefore new since it is not necessary to have recourse to any antioxidant.

It was found that, in the presence of oxygen saturated solutions and solar light, Ticlopidine hydrochloride undergoes an oxidative process, macroscopically appearing a discoloration. This reaction of Ticlopidine hydrochloride oxidation and discoloration is also observed in the presence of some excipients, as it was shown by binary mixtures under stress conditions. The presence of certain commonly used excipients, such as polyvinylpyrrolidone (providone), magnesium stearate, and polyethylene glycols, promotes a discoloration associated to the oxidative degradation of the Ticlopidine hydrochloride. However, the addition of an antioxidant such as citric acid is capable of avoiding this discoloration, even in the presence of these excipients.

Taking as a basis the results of the preliminary tests, the behaviour of the Ticlopidine hydrochloride under the absence of potential agents responsible for the oxidation (such as providone, magnesium stearate, and polyethylene glycols) was studied.

It was concluded that, provided that these agents are not present, it is possible to obtain stable formulations not using any antioxidant.

Thus, the results show that the use of non volatile organic acids, such as citric acid, counteracts the disadvantageous effects of some excipients. However, if these excipients are not included, the inclusion of said antioxidant may also be suppressed.

In order to satisfy the rules of *"Note for Guidance or Inclusion of Antioxidantes and Antimicrobial Preservatives in Medicinal Products"* (CPMP/CVMP/QWP/115/95), it was necessary to develop a technologically feasible and stable formulation not using the above mentioned excipients. Thus, another novelty of this invention is the association on the same formulation of Ticlopedine hydrochloride and alternative excipients having identical functions but being unprovided of oxidative action, such as non metallic lubricants, non polymeric binders and, in the coating, plasticizers not included in the polyethylene glycol group. Another novelty of the present (invention which is also an advantage thereof) is the use of water as the granulating and coating liquid which reduces the process costs, is not dangerous and does not cause environmental contamination.

### EXAMPLES

This invention may be illustrated by the below non limitative examples:

### Example 1 - Preparation of Tablets by Wet Granulation

| **Components** | **Quantitative Composition (mg)** | **Effect** |
|---|---|---|
| Ticlopidine hydrochloride | 250.0 | Active compound |
| Maize starch | 77.8 | Diluent/Binder |
| Microcrystalline cellulose | 43.3 | Diluent |
| Talc | 5.1 | Lubricant |
| Hydrogenated vegetable oil | 3.8 | Lubricant |
| **Total** | **380.0** | |

The Ticlopidine hydrochloride, 80% of the maize starch and all the microcrystalline cellulose are blended together.

A starch decoction is prepared from the remaining starch and is slowly added to the mixture under agitation. The uniformly wetted mixture is granulated through a 1.6 mm opening sieve. The granules are dried at 40°C to a humidity content < 5% and screened through a 1.0 mm opening sieve. At last, the talc and the hydrogenated vegetable oil are added and blended. The obtained granules have rheological and technological properties suitable for the compression and make possible the production of tablets acceptable under the technological and pharmaceutical points of view, particularly as it concerns the uniformity, content and release of the active compound.

### Example 2 - Preparation of Coated Tablets

| **Components** | **Quantitative Composition (mg)** |
|---|---|
| Ticlopidine hydrochloride | 250.000 |
| Maize starch | 54.000 |
| Microcrystalline cellulose | 57.000 |
| Talc | 15.200 |
| Hydrogenated vegetable oil | 3.800 |
| Hydroxypropyl methylcellulose | 3.750 |
| Triacetin | .350 |
| Titanium dioxide | 1.875 |
| **Total** | **386.000** |

The Ticlopidine hydrochloride, 70% of the maize starch and 90% of the microcrystalline cellulose are blended together. The remaining starch is used to prepare a starch decoction which is slowly added to the mixture under agitation. The uniformly wetted mixture is granulated through a 1.6 mm opening sieve. The granules are dried at 50°C to a humidity content < 5% and screened through a 1.0 mm opening sieve. The remaining microcrystalline cellulose, the talc and the hydrogenated vegetable oil are added and mixed; the granules are compressed. At last, the cores are coated by application of an aqueous suspension containing the hydroxypropyl methylcellulose, triacetin and titanium dioxide. The intermediate products and the coated tablets are acceptable under the technological and pharmaceutical points of view, particularly as it concerns the uniformity, content and release of the active compound.

### Example 3 - Preparation of Ticlopidine Hydrochloride Tablets

| **Components** | **Quantitative Composition (mg)** | **Function** |
|---|---|---|
| Ticlopidine hydrochloride | 250.0 | Active compound |
| Maize starch | 67.7 | Diluent/Binder |
| Microcrystalline cellulose | 43.3 | Diluent |
| Hydrogenated vegetable oil | 19.0 | Lubricant |
| **Total** | **380.0** | |

The Ticlopidine hydrochloride, 80% of the maize starch and all the microcrystalline cellulose are blended together. A starch decoction is prepared from the remaining starch and slowly added to the mixture under agitation. The granules are dried at 40°C to a humidity content < 5% and screened through a 1.0. mm opening sieve. At last, the hydrogenated vegetable oil is added and blended. The thus obtained granules have rheological and technological properties suitable for the compression and make possible the production of tablets acceptable under the technological and pharmaceutical points of view, particularly as it concerns the uniformity, content and release of the active compound.

### Example 4 - Preparation of Coated Tablets

| **Components** | **Quantitative Composition (mg)** |
|---|---|
| Ticlopidine hydrochloride | 250.000 |
| Maize starch | 67.700 |
| Microcrystalline cellulose | 43.300 |
| Hydrogenated vegetable oil | 19.300 |
| Hydroxypropyl methylcellulose | 3.750 |
| Triacetin | .375 |
| Titanium dioxide | 1.875 |
| **Total** | **386.000** |

The Ticlopidine hydrochloride, 70% of the maize starch and 90% of the microcrystalline cellulose are blended together. The remaining starch is used to prepare a starch decoction and this is slowly added to the mixture under agitation. The uniformly wetted mixture is granulated through a 1.6 mm opening sieve. The granules are dried at 50°C to a humidity content < 5% and screened through a 1.0 mm opening sieve. The remaining microcrystalline cellulose, the talc and the hydrogenated vegetable oil are added and blended; the granules are compressed. At last, the cores are coated by applying an aqueous suspension containing the hydroxypropyl methylcellulose, triacetin and titanium dioxide. The intermediate products and the coated tablets are acceptable under the technological and pharmaceutical points of view, particularly as it concerns the uniformity, content and release of the active compound.

The coated tablets, prepared according to Examples 2 and 4, were packed in a suitable packaging and their stability tested. The obtained results show that it is possible to obtain stable Ticlopidine hydrochloride formulations free of any antioxidant.

| 40°C/ 75% RH | | |
|---|---|---|
| Time (month) | Dosage (%) | Dissolution (%) |
| 0 | 98.9 | 101 |
| 2 | 96.9 | 105 |
| 3 | 98.6 | 110 |
| 4 | 98.2 | 103 |
| 5 | 96.3 | - |
| 6 | 98.1 | 100 |

### REFERENCES

1. Note for Guidance of Inclusion of Antioxidants and Antimicrobial Preservatives in Medicinal Products.
   CPMP/CVMP/QWP/115/95
2. An Overview of Antioxidant, Preservative and Solvent Excipients Used in the Pharmaceutical Industry.
3. The Theory and Practice of Industrial Pharmacy, 780-784.
4. Florence, A.T.; Attwood, D (1988). Physicochemical Principles of Pharmacy. 2nd Edtion, 420-426.

## Claims

1. A pharmaceutical composition containing a thienopyridine derivative acid salt, characterized in that said composition is stable though being free of any antioxidant and/or due to the absence of any agent inducing the Thienopyridine derivative acid salt degradation.

2. The pharmaceutical composition as claimed in claim 1, characterized in that 5-(2-chlorobenzyl)-4,5,6,7-tetrahydrothieno[3,2-c]pyridine hydrochloride (Ticlopidine hydrocloride) is used as a thienopyridine derivative.

3. The pharmaceutical composition as claimed in claims 1 and 2, characterized in that it contains an excipient comprising one or several diluents, one or several. binding agents, one or several disintegrants and one or several lubricant agents.

4. The pharmaceutical composition as claimed in claim 3, characterized in that, as a diluent, it contains microcrystalline cellulose, maize starch, lactose, or a mixture thereof.

5. The pharmaceutical composition as claimed in claim 3, characterized in that it contains, as a binding agent, maize starch, a cellulose derivative or a polysaccharide gum, or a mixture thereof.

6. The pharmaceutical composition as claimed in claim 3, characterized in that it contains, as a disintegrant, maize starch, a starch derivative such as sodium starch glycolate, or crosslinked carboxymethyl cellulose sodium, or a mixture thereof.

7. The pharmaceutical composition as claimed in claim 3, characterized in that it contains, as a lubricant, a hydrogenated vegetable oil, talc or anhydrous colloidal silica, or a mixture thereof.

8. The pharmaceutical composition as claimed in any one of previous claims, characterized in that the administration form is a tablet or a capsule for immediate or retarded release of the active compound.

9. The pharmaceutical composition as claimed in any one of the claims 1 to 8, wherein the administration form is a coated tablet, the coating containing a polymeric agent, a plasticizer, an opacifier and a coloring agent.

10. The pharmaceutical composition as claimed in claim 10, characterized in that it contains, as a polymer, a cellulose derivative such as hydroxypropyl methylcellulose, a methacrylic acid derivative, or a mixture thereof.

11. The pharmaceutical composition as claimed in claim 10, characterized in that it contains, as a plasticizer, triacetin, dibutyl sebacate, dibutyl phtalate, or a mixture thereof.

12. The pharmaceutical composition as claimed in claim 10, characterized in that it contains, as an opacifier, titanium dioxide.

13. The pharmaceutical composition as claimed in claim 10, characterized in that it contain, one or several of legally approved colorants to be used in medicinal products.

14. Process to prepare pharmaceutical compositions as claimed in any one of the previous claims.
